# EUROPEAN PATENT APPLICATION

(11) **EP 3 831 401 A1**
(43) Date of publication of application: **09.06.2021**
(21) Application number: 19821864.6
(22) Date of filing: 20.06.2019
(51) Int. Cl.: A61K 38/17, A61K 39/35, A61P 37/08

(54) **RECOMBINANT DUST MITE ALLERGEN PROTEIN DRUG MIXTURE AND USE THEREOF**

(30) Priority: 22.06.2018 CN 201810647416
(71) Applicant: Zonhon Biopharma Institute, Inc., Changzhou, Jiangsu 213125 (CN)
(72) Inventor: MA, Bruce Yong, Changzhou, Jiangsu 213125 (CN); FAN, Yu, Changzhou, Jiangsu 213125 (CN); WU, Cheng, Changzhou, Jiangsu 213125 (CN); ZHAO, Lili, Changzhou, Jiangsu 213125 (CN); JIANG, Chenyang, ChangZhou, Jiangsu 213125 (CN)
(74) Representative: Lederer & Keller Patentanwälte Partnerschaft mbB
(86) International application number: PCT/CN2019/091968
(87) International publication number: WO 2019/242662

(57) **Abstract**

Disclosed is a recombinant dust mite allergen protein composition, comprising a recombinant dust mite type I allergen DP1 and DF1 proteins and a recombinant dust mite type II allergen DP2 and DF2 proteins, wherein same are respectively mixed in an activity ratio of 1:1:1:1 or mixed in a mass ratio of (1-5):(1-20):(1-10):(1-20). Compared to existing products, (1) the recombinant allergens have the advantages of a high purity and a controllable quality, etc.; (2) the recombinant proteins have better specificity; and (3) compared with the natural extract, the composition has better immunogenicity, reduces the risk of serious allergic side reactions and improves the effect of a desensitization treatment. In addition, the recombinant allergen composition of the present invention not only has a better activity than the natural allergen protein in the same proportions, but also has a higher activity than the corresponding natural single component when compared with the single component.

SEQUENCE LISTING

<110>  ½­ËÕÕÚ°ìÉúÎï¹¤³Ì´´Ò©ÑÐ¾¿Ô°ÓÐÏÞ¹«Ë¾

<120>  ÖØ×é³¾òý±äÓ¦Ô­µ°°×Ò©Îï°Ï¼Á¼°ÆäÓ¦ÓÃ


<160>  6

<170>  PatentIn version 3.3

<210>  1
<211>  912
<212>  DNA
<213>  Dermatophagoides pteronyssinus

<400>  1
agaccatcct ccatcaagac tttcgaagag tacaagaagg ctttcaacaa gtcctacgct      60

actttcgagg acgaagaggc tgctagaaag aacttcttgg aatccgttaa gtacgttcag     120

tccaacggtg gtgctatcaa ccacttgtct gacttgtctt tggacgagtt caagaacaga     180

ttcttgatgt ccgctgaagc tttcgagcac ttgaaaacac agttcgactt gaacgctgaa     240

actaacgctt gttccatcaa cggtaacgct ccagctgaaa tcgacttgag acagatgaga     300

actgttactc caatcagaat gcagggtggt tgtggttctt gttgggcttt ttctggtgtt     360

gctgctactg aatccgctta cttggcttac agacaacagt ccttggactt ggctgaacaa     420

gagttggttg actgtgcttc ccaacatggt tgtcacggtg acactattcc aagaggtatc     480

gagtacatcc agcacaacgg tgttgttcaa gaatcctact acagatacgt tgctagagag     540

cagtcctgta gaagaccaaa cgctcaaaga ttcggtatct ccaactactg tcagatctac     600

ccacctaacg ctaacaagat cagagaggct ttggctcaaa ctcactccgc tatcgctgtt     660

atcatcggta tcaaggactt ggacgctttc agacactacg acggtagaac tatcatccag     720

agagacaacg gttaccagcc aaactaccac gctgttaata tcgttggtta ctctaacgct     780

caaggtgttg actactggat cgttagaaac tcctgggaca ctaactgggg tgataacggt     840

tacggttact tcgctgctaa cattgacttg atgatgattg aagagtaccc atacgttgtt     900

atcttgcatt aa                                                         912


<210>  2
<211>  912
<212>  DNA
<213>  Dermatophagoides farinae

<400>  2
agaccagcat ctatcaagac atttgaagag ttcaagaaag cttttaacaa gaactacgcc      60

actgttgaag aggaagaggt cgctagaaag aactttttgg aatctttgaa gtatgttgag     120

gcaaacaaag gtgctattaa tcacttgtct gatttgtccc ttgacgaatt caagaacaga     180

tacttgatga gtgctgaagc ctttgagcaa ttgaaaacac agttcgacct taacgccgaa     240

acctcagcat gtagaattaa ctcagttaat gtccctagtg agttggatct tagaagtttg 300

agaaccgtta ctcctattag aatgcaaggt ggatgtggtt cttgctgggc cttctccgga 360

gttgcagcta ccgaatctgc ttacttggcc tacagacaga cttcattgga tcttagtgaa 420

caagagttgg ttgactgtgc ttcccagcat ggttgccacg gagacactat tcctagaggt 480

attgaataca tccaacagaa cggagttgtc gaagagagat catacccata tgttgctaga 540

gagcaacagt gtagaagacc taactcacaa cactacggta ttagtaacta ctgccagatc 600

tatccacctg atgttaagca aattagagaa gcattgacac agacccatac tgcaattgct 660

gtcattatcg gaatcaagga tttgagagct ttccaacatt acgacggtag aacaattatc 720

caacacgata acggatacca gccaaattat catgctgtta acattgtcgg ttacggatcc 780

acccaaggtg ttgattattg gatcgtcaga aactcttggg atactacatg gggtgattct 840

ggttacggat atttccaagc tggaaacaat ttgatgatga ttgaacagta cccttatgtt 900

gtcatcatgt aa 912

<210> 3
<211> 441
<212> DNA
<213> Dermatophagoides pteronyssinus

<400> 3
atgatgtaca agatcttgtg tttgtctttg ttggttgctg ctgttgctag agaccaggtt 60

gacgttaagg actgtgctaa ccacgagatc aagaaggttt tggttccagg ttgtcacggt 120

tccgagccat gtattattca cagaggtaag ccattccagt tggaggctgt tttcgaagct 180

aaccagaaca ctaagactgc taagattgag atcaaggctt ccatcgacgg tttggaagtt 240

gacgttccag gtattgaccc aaacgcttgt cactacatga agtgtccatt ggttaagggt 300

cagcagtacg acatcaagta cacttggaat gttccaaaga tcgctccaaa gtccgagaac 360

gttgttgtta ctgttaaggt tatgggtgac gacggtgttt tggcttgtgc tattgctact 420

cacgctaaga tcagagacta a 441

<210> 4
<211> 444
<212> DNA
<213> Dermatophagoides farinae

<400> 4
atgatctcta agatcttgtg tttgtctttg ttggttgctg ctgttgttgc tgaccaggtt 60

gacgttaagg actgtgctaa caacgagatc aagaaagtta tggttgacgg ttgtcacggt 120

tccgacccat gtattattca cagaggtaag ccattcactt tggaggcttt gttcgacgct 180

aaccagaaca ctaagactgc taagatcgag attaaggctt ccttggacgg tttggagatt 240

gacgttccag gtatcgacac taacgcttgt cactttatga agtgtccatt ggttaagggt 300

cagcagtacg acatcaagta cacttggaat gttccaaaga tcgctccaaa gtccgagaac 360

gttgttgtta ctgttaagtt gatcggtgac aacggtgttt tggcttgtgc tattgctact 420

cacggtaaga tcagagatta atag 444

<210> 5
<211> 18
<212> DNA
<213> ÈË¹¤ÐòÁÐ

<400> 5
tgtaaaacga cggccagt 18

<210> 6
<211> 17
<212> DNA
<213> ÈË¹¤ÐòÁÐ

<400> 6
caggaaacag ctatgac 17

## Description

### TECHNICAL FIELD

The present invention relates to a drug composition for treating dust mite allergy, and in particular relates to an improved recombinant dust mite allergen protein pharmaceutical composition and use thereof.

**BACKGROUND OF THE INVENTION** Among many allergens that cause allergic diseases, dust mites are the most important allergen. In the specific immunodiagnosis,the positive rate of dust mites in patients with allergic diseases is about 70-80%. There are many kinds of dust mites, which are widely present in human life and work environment. The excreta, metabolites and bodies of dust mites have strong allergenicity. According to statistics, about 10% of the global population is allergic to dust mites, and about 80% of extrinsic asthma is caused by dust mites.

Currently, dust mite allergen extracts are mainly used clinically to treat dust mite allergy. For example, the "Changdi" Dermatophagoides farinae drops of Zhejiang Wolwo pharma, which was launched in 2006, is the extract of a *Dermatophagoides farinae* metabolism culture; Actair *Dermatophagoides pteronyssinus* sublingual tablets launched in Japan in 2015 are used to treat allergic rhinitis caused by *Dermatophagoides pteronyssinus* (or referred to as house dust mite), and also include four components of natural types I and II house dust mites and types I and II *Dermatophagoides farinaes;* and Odactra, which was approved by the FDA in 2017, also includes four components of natural type I and type II house dust mites and type I and type II *Dermatophagoides farinaes.* It shows that the use of mixtures has become a trend in dust mite desensitization treatment.

However, the composition of the natural allergen extract is very complex, and is very difficult to maintain its components. The natural allergen extract is easy to be contaminated by exogenous toxic substances and pathogenic microorganisms, which affects its repeatability and safety. Long-term use in the treatment can easily lead to local reactions such as redness, swelling, induration and necrosis, and systemic reactions such as shock, edema, bronchospasm, urticaria, angioedema and systemic erythema. In addition, the use of the crude extract for diagnosis cannot clarify the degree of reaction of the patient to the components of the allergens, which may easily lead to misdiagnosis. The quality of allergens is very important for the diagnosis and treatment of allergic reaction diseases. The allergens used for immunological diagnosis and treatment should be pure products rather than crude extracts. In addition, dust mite allergens mainly exist in excreta and mite bodies. The extraction method is time-consuming, cumbersome and costly, and side reactions in immunotherapy cannot be avoided.

### SUMMARY OF THE INVENTION

In order to overcome the above shortcomings, the objective of the present invention is to provide a recombinant dust mite allergen protein pharmaceutical composition, and optimize the pharmaceutical composition ratio to obtain the best activity.

First, the present invention provides a recombinant dust mite allergen protein pharmaceutical composition, including recombinant dust mite type I allergen DP1 and DF1 proteins and recombinant dust mite type II allergen DP2 and DF2 proteins, wherein the four types of dust mite allergen proteins DP1:DP2:DF1 :DF2 are respectively mixed in an activity ratio of 1:1:1:1 or mixed in a mass ratio of (1-5):(1-20):(1-10):(1-20).

Preferably, the four types of dust mite allergen proteins DP1:DP2:DF1:DF2 in the pharmaceutical composition are respectively mixed in a mass ratio of 1:(1-20):1:(1-20).

More preferably, the four types of dust mite allergen proteins in the pharmaceutical composition are mixed in a mass ratio of 1:5:1:5.

Preferably, the recombinant dust mite type I allergen DP1 and DF1 proteins and the recombinant dust mite type II allergen DP2 and DF2 proteins are respectively obtained by expressing the genes as shown in SEQ ID NO: 1, SEQ ID NO: 2, SEQ ID NO: 3 and SEQ ID NO: 4 through a *Pichia pastoris* expression system.

More preferably, the recombinant dust mite type I allergen protein is prepared by the following method:
1. The above gene is constructed on a plasmid pPICZα, the plasmid is transfected into *Pichia pastoris* X33 competent cells, and the cells are cultured to obtain the host monoclonal engineered bacteria; and 2. The monoclonal engineered bacteria are selected and subjected to high-density fermentation, and the recombinant dust mite type I allergen protein is obtained by three steps of purification of ion exchange chromatography, anion exchange chromatography, and hydrophobic chromatography.

Preferably, in the high-density fermentation process, the main fermentation process for expressing the recombinant dust mite type I allergen DP1 and DF1 proteins is as follows: the fermentation medium used is a 40% BSM medium; at the induction stage of high-density fermentation, the induction temperature is 25-27°C, and the pH value is 6.5±0.2; at the induction stage of high-density fermentation, the feeding rate of methanol is 30 mL/h⁻¹L⁻¹, and DO is maintained not higher than 40%; at the proliferation stage of bacteria during high-density fermentation, the fermentation temperature is 27°C, the pH is 5.5±0.2, the rotation speed is 300 rpm, the DO value is 100%, and PTM1 (2.4 ml/L) is added; and at the start of the rate-limited growth stage of high-density fermentation, 50% glycerol is fed at a rate of 30 ml/h⁻¹L⁻¹, and then the feeding rate is increased to 60 ml/h⁻¹L⁻¹.

Preferably, when the recombinant dust mite type I allergen DP1 and DF1 proteins are expressed, in a three-step purification process after high-density fermentation, the purification packing for cation exchange chromatography is SP FF, and the preferred pH range is 4.0-6.5. More preferably, the pH of the sample and buffer in the cation exchange chromatography is 5.0.

The purification packing for the anion exchange chromatography is Q FF, the preferred pH range is 6.0-8.0, and the preferred conductivity is 2.0 mS/cm-20.0 mS/cm. More preferably, the pH of the sample and buffer in the anion exchange chromatography is 8.0, and the conductivity is 20.0 mS/cm.

The purification packing for the hydrophobic chromatography is phenyl FF, the preferred pH range is 6.5-8.5, and the preferred concentration of ammonium sulfate is 1.0-2.0 M. More preferably, the concentration of ammonium sulfate in the sample and equilibration buffer in the hydrophobic chromatography is 2.0 M, and the pH is 8.5.

Preferably, in the high-density fermentation process, the main fermentation process for expressing the recombinant dust mite type II allergen DP2 and DF2 proteins is as follows: the fermentation medium used is a 60% BSM medium; at the induction stage of high-density fermentation, the induction temperature is 20-22°C, and the pH value is 6.0±0.2; at the induction stage of high-density fermentation, the feeding rate of methanol is 30 mL/h⁻¹L⁻¹, and DO is maintained not higher than 40%; and at the start of the rate-limited growth stage of high-density fermentation, 50% glycerol is fed at a rate of 30 ml/h⁻¹L⁻¹, and then the feeding rate is increased to 60 ml/h⁻¹L⁻¹.

Preferably, when the recombinant dust mite type II allergen DP2 and DF2 proteins are expressed, in a three-step purification process after high-density fermentation, the purification packing for cation exchange chromatography is SP FF, and the preferred pH range is 4.0-6.0. More preferably, the pH of the sample and buffer is 6.0.

The purification packing for the anion exchange chromatography is Q FF, the preferred pH range is 6.0-9.0, and the preferred conductivity is 2.0 mS/cm-20.0 mS/cm. More preferably, the pH of the sample and buffer is 7.5, and the conductivity is 10.0 mS/cm.

The purification packing for the hydrophobic chromatography is phenyl FF, the preferred pH range is 6.0-8.0, and the preferred concentration of ammonium sulfate is 1.0-2.0 M. More preferably, the concentration of ammonium sulfate in the sample and equilibration buffer is 1.0 M, and the pH is 6.0.

Compared with the existing products, the present invention has the following advantages: firstly, compared with a crude extract: (1) the recombinant allergen has a higher purity, and does not contain non-allergenic components, enzymes, enzyme inhibitors, toxic proteins and the like compared with the crude extract; (2) the recombinant protein has good specificity; the components in the crude extract are complex, patients may only react with some of the components, and the specificity is poor; while the recombinant allergen has a single component and good specificity; and (3) the recombinant allergen can replace the natural extract in activity, reduces IgE-bound antigenic epitopes compared with the natural extract, effectively reduces IgE-mediated allergic reactions, while retains the necessary domains for allergen T cell recognition, has good immunogenicity, reduces the risk of immunotherapy, and improves the effect of desensitization treatment. Secondly, a most suitable composition ratio is found after repeated exploration by the applicant as the recombinant allergen protein pharmaceutical composition of the present invention, so that the composition not only has better activity than the natural allergen protein in the same proportions (the activity of the recombinant allergen protein alone is basically the same as that of the natural allergen protein), but also has a higher activity than that of the most active allergen protein compared with a single component.

### BRIEF DESCRIPTION OF THE DRAWINGS

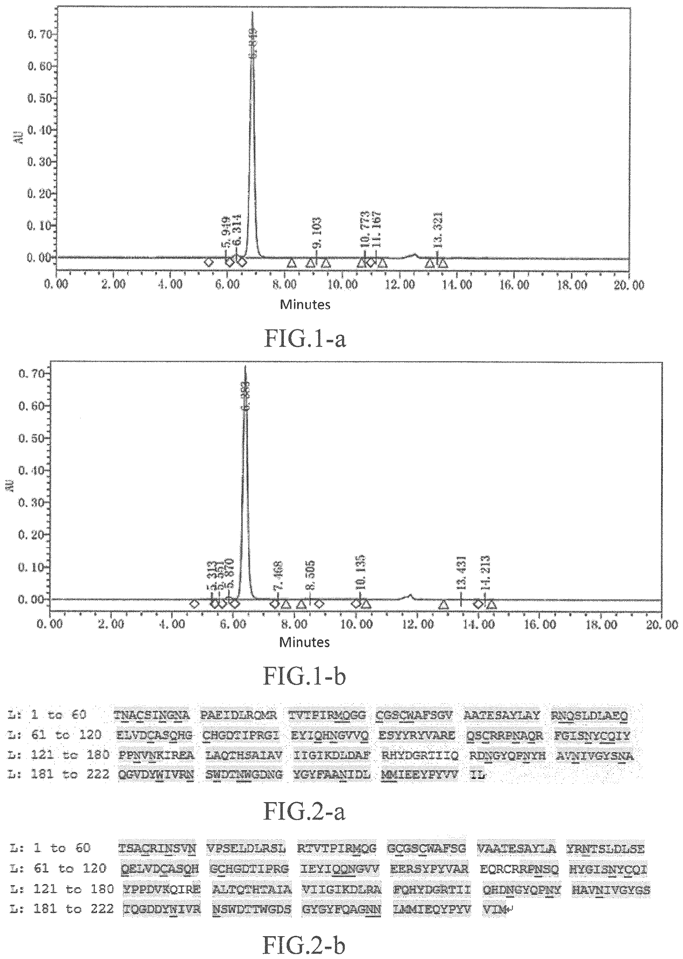

Figure 1 shows the HPLC analysis results of the purity of DP1/DF1 proteins.
Figure 1-a shows the RP-HPLC analysis result of the DP1 protein; Figure 1-b shows the RP-HPLC analysis result of the DF1 protein.
Figure 2 shows the analysis results of the amino acid coverage of the DP1/DF1 proteins.
Figure 2-a shows the amino acid coverage of the DP1 protein; Figure 2-b shows the amino acid coverage of the DF1 protein.

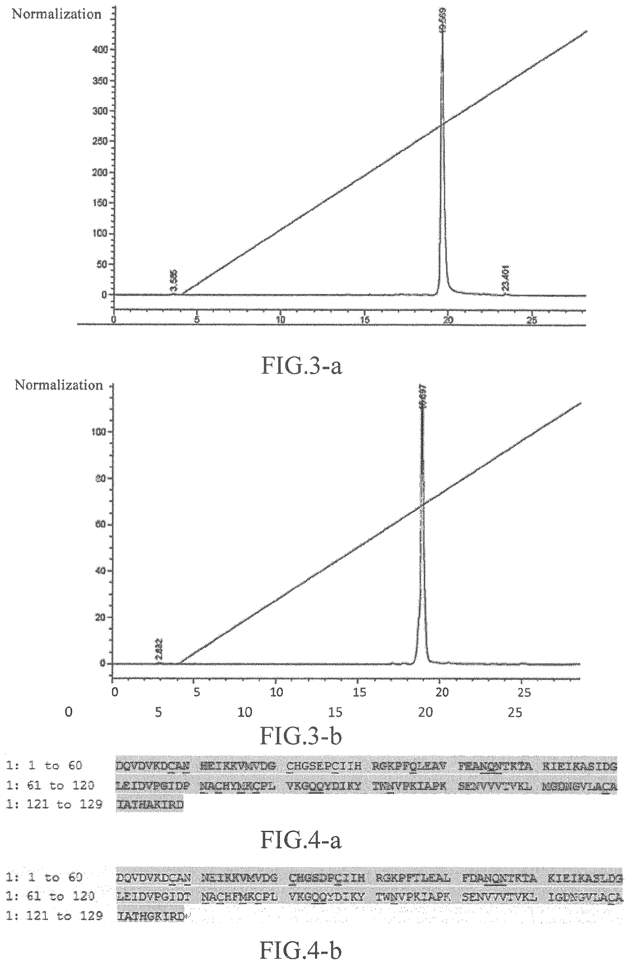

Figure 3 shows the HPLC analysis results of the purity of DP2/DF2 proteins.
Figure 3-a shows the RP-HPLC analysis result of the DP2 protein; Figure 3-b shows the RP-HPLC analysis result of the DF2 protein.
Figure 4 shows the analysis results of the amino acid coverage of the DP2/DF2 proteins.
Figure 4-a shows the analysis result of the amino acid coverage of the DP2 protein; Figure 4-b shows the analysis result of the amino acid coverage of the DF2 protein.

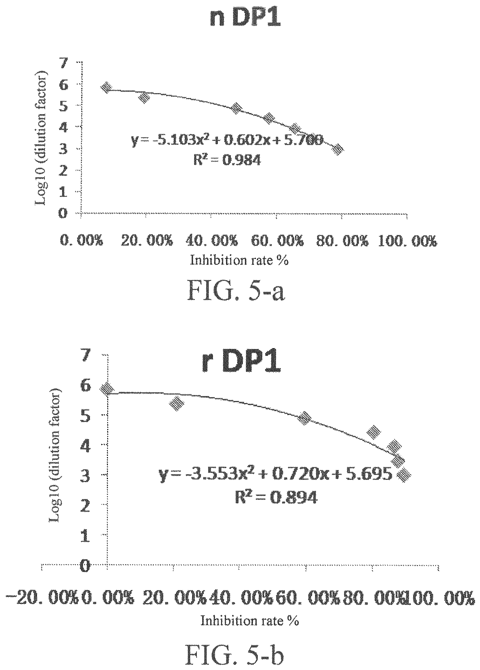

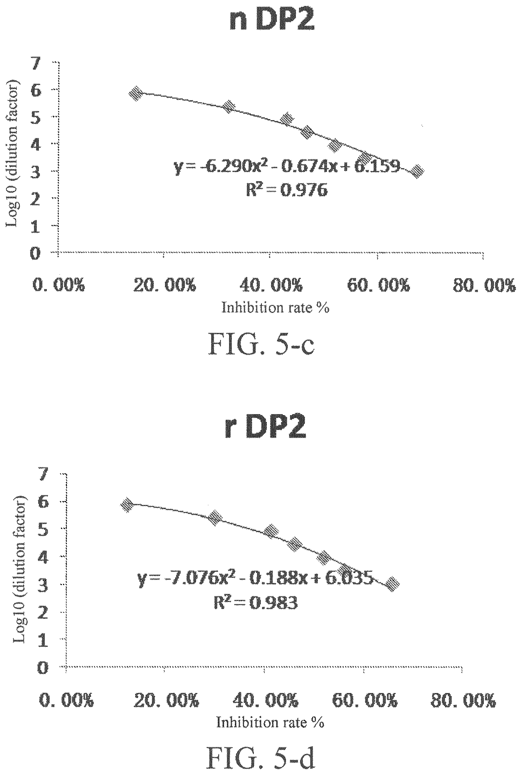

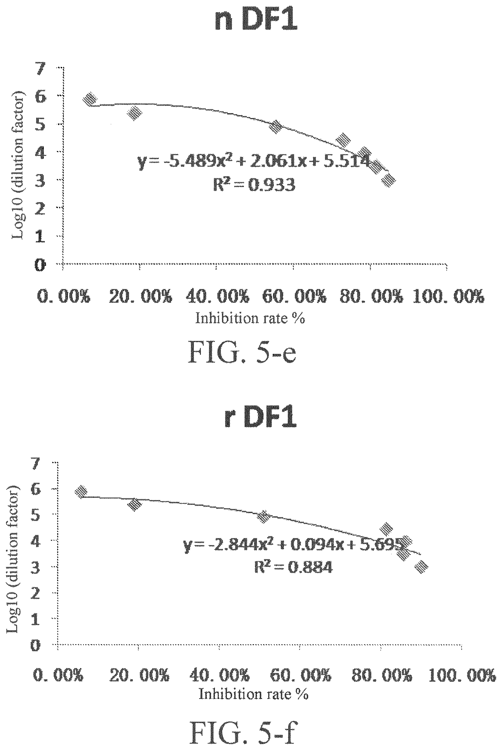

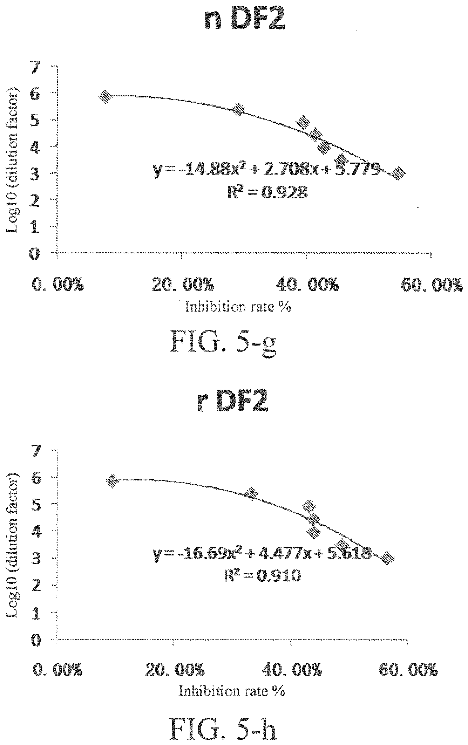

Figure 5 is a curve diagram showing the relationship between the Log10 (dilution factor) of single-component recombinant protein (r) and natural protein (n) and the inhibition rate.
Figure 5-a is a curve diagram showing the relationship between the LoglO (dilution factor) of natural DP1 protein and the inhibition rate.
Figure 5-b is a curve diagram showing the relationship between the LoglO (dilution factor) of recombinant DP1 protein and the inhibition rate.
Figure 5-c is a curve diagram showing the relationship between the Log10 (dilution factor) of natural DP2 protein and the inhibition rate.
Figure 5-d is a curve diagram showing the relationship between the Log10 (dilution factor) of recombinant DP2 protein and the inhibition rate.
Figure 5-e is a curve diagram showing the relationship between the Log10 (dilution factor) of natural DF1 protein and the inhibition rate.
Figure 5-f is a curve diagram showing the relationship between the Log10 (dilution factor) of recombinant DF1 protein and the inhibition rate.
Figure 5-g is a curve diagram showing the relationship between the Log10 (dilution factor) of natural DF2 protein and the inhibition rate.
Figure 5-h is a curve diagram showing the relationship between the Log10 (dilution factor) of recombinant DF2 protein and the inhibition rate.

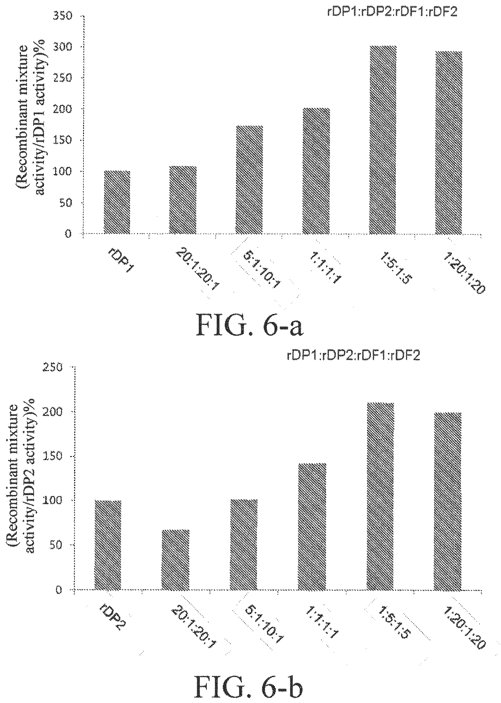

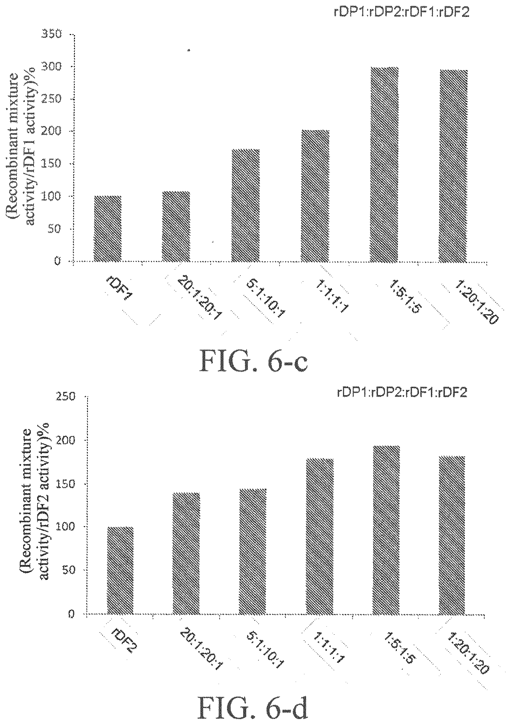

Figure 6 shows the percentage of activity when the recombinant composition in different ratios (mass ratio) are compared with the single-component recombinant protein.
Figure 6-a shows the comparison of activity between the recombinant composition in different ratios (mass ratio) and the single-component rDP1.
Figure 6-b shows the comparison of activity between the recombinant composition in different ratios (mass ratio) and the single-component rDP2.
Figure 6-c shows the comparison of activity between the recombinant composition in different ratios (mass ratio) and the single-component rDF1.
Figure 6-d shows the comparison of activity between the recombinant composition in different ratios (mass ratio) and the single-component rDF2.

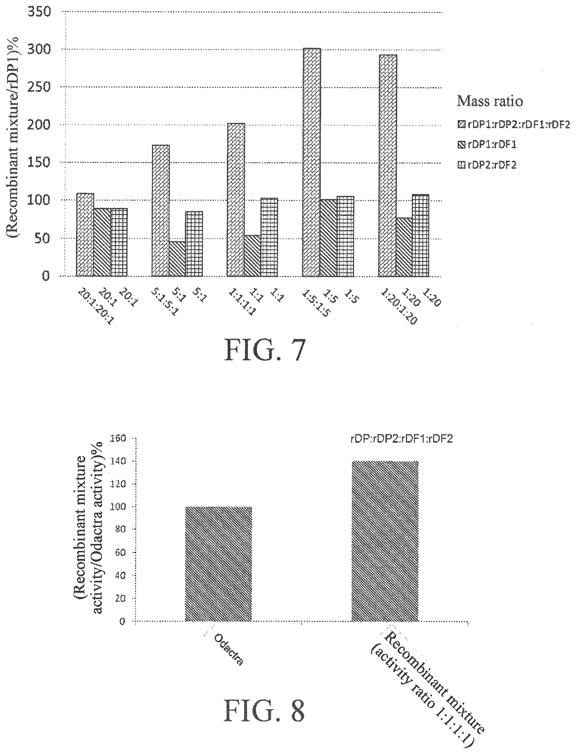

Figure 7 shows the comparison of activity between the recombinant composition of four types of proteins in different ratios (mass ratio) and two allergens of types I and II.
Figure 8 shows the comparison of activity between the recombinant mixture and Odactra with the same activity.

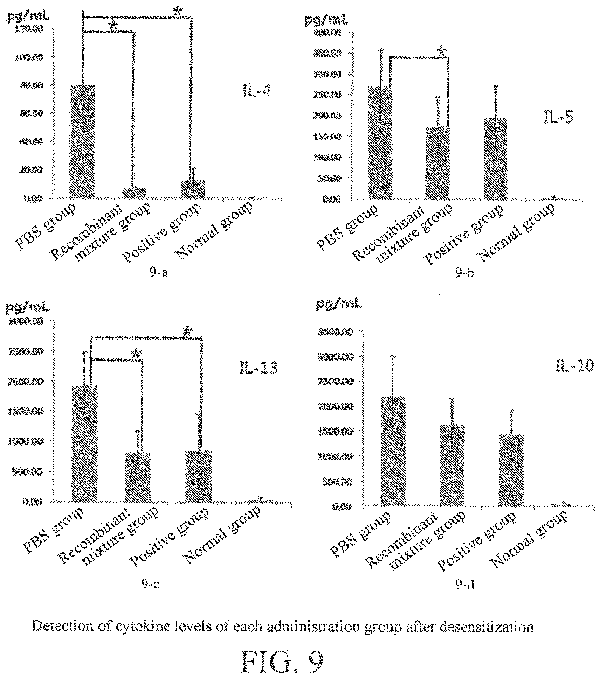

Figure 9 shows the detection results of the cytokine content in the supernatant of spleen cell stimulation of each administration group after desensitization.
Figure 9-a shows the detection results of the IL-4 content in the supernatant of spleen cell stimulation of each administration group after desensitization.
Figure 9-b shows the detection results of the IL-5 content in the supernatant of spleen cell stimulation of each administration group after desensitization.
Figure 9-c shows the detection results of the IL-13 content in the supernatant of spleen cell stimulation of each administration group after desensitization.
Figure 9-d shows the detection results of the IL-10 content in the supernatant of spleen cell stimulation of each administration group after desensitization.

### DETAILED DESCRIPTION

The present invention will be further described below in conjunction with specific Example s. It should be understood that the cited example s are only used to illustrate the present invention and not to limit the scope of the present invention.

### Example 1 Recombinant proDP1 codon optimization modification, expression plasmid construction and host engineering strain construction

### Step 1: recombinant codon optimization modification

According to the DNA sequence of DP1 (ENA accession number: FM177224.1) published by EMBL-EBI, by combining the requirements of the expression system of the present invention and performing verifications for many times, the inventor obtained the codon optimized and modified proDP1 gene of the present invention (the gene is obtained by adding an alpha-factor signal peptide and a Kozak sequence GCCACCATGG to the wild-type gene, and is codon optimized), and the nucleotide sequence is shown in SEQ ID NO: 1.

According to the DNA sequence of DF1 (GenBank accession number: AB034946.1) published by GenBank, by combining the requirements of the expression system of the present invention and performing verifications for many times, the inventor obtained the codon optimized and modified proDFl gene of the present invention (the gene is obtained by adding an alpha-factor signal peptide and a Kozak sequence GCCACCATGG to the wild-type gene, and is codon optimized), and the nucleotide sequence is shown in SEQ ID NO:2.

According to the DNA sequence of DP2 (GenBank accession number: AAF86462) published by GenBank, by combining the requirements of the expression system of the present invention and performing verifications for many times, the inventor obtained the codon optimized and modified DP2 gene of the present invention, and the nucleotide sequence is shown in SEQ ID NO: 3.

According to the DNA sequence of DF2 (GenBank accession number: EF139432.1) published by GenBank, by combining the requirements of the expression system of the present invention and performing verifications for many times, the inventor obtained the DF2 gene of the present invention after the gene is codon optimized, and the nucleotide sequence is shown in SEQ ID NO: 4.

### Step 2: construction of expression plasmid containing proDP1 gene

The XhoI enzymatic restriction site sequence is introduced at the 5' end of the codon optimized proDP1, the XbaI enzymatic restriction site sequence is introduced at the 3' end, and whole gene synthesis is performed. The synthesized gene fragment is constructed into the pUC57 plasmid (provided by Nanjing GenScript Biotechnology Corporation) to obtain a long-term preservation plasmid, denoted as pUC57-proDP1 plasmid.

The pUC57-proDPl plasmid is used as a template to perform PCR amplification, and the primer sequences used are as follows:
Forward primer (SEQ ID No: 5):
   M13F: TGTAAAACGACGGCCAGT
Reverse primer (SEQ ID NO: 6):
   M13R: CAGGAAACAGCTATGAC

The total reaction volume is 50 µL, wherein 2.5 µL of each of the primers at a concentration of 10 µmol/L, 1 µL of dNTP at a concentration of 10 mmol/L, and 0.5 µL of DNA polymerase Q5 (#M0491L, purchased from New England BioLabs) at 2 U/µL, are added. The reaction is performed at 98°C for 5 s, 55°C for 45 s, and 72°C for 30 s. After 25 cycles, the product is analyzed by 1.0% agarose gel electrophoresis, and the result shows that the product size is consistent with the expected size (1000 bp). After double enzymatic digestion with XhoI (#R0146S, purchased from New England BioLabs) and XbaI (#R01445S, purchased from New England BioLabs) respectively, 1% agarose electrophoresis is performed, and the gene product obtained is purified with a DNA gel recovery kit (DP214, purchased from Tiangen Biotech (Beijing) Co., Ltd.). A T4 ligase (#M0202S, purchased from New England BioLabs) is used to ligate the gene product into pPICZaA plasmid (V173-20, purchased from Invitrogen). The plasmid is transformed into DH5α competent cells (CB101, purchased from Tiangen Biotech (Beijing) Co., Ltd.), and the cells are cultured overnight at 37°C in an LB solid medium containing bleomycin (purchased from Invitrogen). On the second day, positive clonal bacteria are selected, sequenced and compared. If the sequence is completely consistent with the expected sequence, the proDP1 codon optimized expression plasmid is obtained, denoted as pPICZα-proDP1.

### Step 3: construction of Pichia pastoris host engineering strain containing recombinant proDP1 gene

Prepare a YPDS solid medium according to the instruction of an Easy SelectPichia Expression Kit of Invitrogen, containing yeast extract 10 g/L, peptone 20 g/L, glucose 20 g/L, agarose 15 g/L, and sorbitol 182 g/L.

Electrocompetent cells are prepared according to the instruction of the Easy SelectPichia Expression Kit of Invitrogen. The plasmid pPICZα-proDP1 obtained in step 2 is enzymatically digested and linearized with SacI restriction enzyme (#R0156S, purchased from New England Biolabs). After ethanol precipitation, a linearized vector is electrotransformed into *Pichia pastoris* X33 competent cells. The cells are spread on a YPDS solid medium and cultured at 30°C until a transformant grows.

The construction of expression plasmids and host engineering strains of proDF1, DP2 and DF2 are consistent with the above methods, and the description will not be repeated here.

### Example 2 Large-scale (30 L) high-density fermentation experiment of recombinant DP1/DF1

First, large-scale (30 L) high-density fermentation of DPI is explained as follows.

### Step 1: activation of recombinant strain

Glycerol bacterial seeds obtained by the above example and cryopreserved in a working seed bank at -80°C are taken and streaked on a YPD solid medium (yeast extract 10 g/L, peptone 20 g/L, glucose 20 g/L, and agarose 15 g/L), and cultured in a constant temperature and constant humidity box at 30°C for 3-5 days.

### Step 2: primary seed solution culture

The monoclonal colonies on the plate in step 1 are picked and cultured in a YPD liquid medium (yeast extract 10 g/L, peptone 20 g/L, and glucose 20 g/L) at 30°C and 220 rpm to OD₆₀₀≈6.0. The bacteria are observed under a microscope to ensure that there is no other bacteria, thus, the primary seed solution for fermentation is obtained.

### Step 3: secondary seed solution culture

The primary seed solution obtained in step 2 is inoculated into a Sartorius B-plus fermentation tank, and incubated in 60% BSM, at pH=5.5±0.2, 27°C, 300rpm. Through the control of ventilation and rotation speed conditions, the dissolved oxygen is kept at 25%, until finally OD₆₀₀≈40, and the wet weight reaches about 80 g/L. The bacteria are observed under a microscope to ensure that there is no other bacteria, thus, the secondary seed solution for fermentation is obtained.

### Step 4: fermentation process

A Sartorius Cplus bioreactor is cleaned. A pH meter probe of the fermentation tank is calibrated with standard solutions of pH=7.0 and pH=4.0 respectively. Then, according to the designed interval of key process parameters in example 1, it is determined that the fermentation medium is 40% BSM, and 20 L of fermentation medium is prepared and poured into the fermentation tank. The fermentation medium is sterilized online at 121°C for 20 min. After the temperature drops to 50°C, an adjustment is performed with concentrated ammonia so that pH=5.5±0.2.

The seed solution obtained in step 3 is inoculated into the fermentation tank at a ratio of 1:15 (V/V, seed solution/fermentation medium). At the initial proliferation stage of recombinant *Pichia pastoris,* the fermentation temperature is 27°C, pH=5.5±0.2, the rotation speed is 300 rpm, the DO value is 100%, and PTM1 (2.4 ml/L) is added. The initial proliferation stage lasts for about 20 h. When the carbon source is exhausted, the dissolved oxygen value rises rapidly, and the wet weight of the bacteria reaches 100 g/L. At this time, the bacteria enter a rate-limited growth stage. In the first 2 h of the stage, 50% glycerol is added at a rate of 30 ml/h⁻¹L⁻¹. After 2 h, the feeding rate is increased to 60 ml/h⁻¹L⁻¹. After feeding for 4 h, the wet weight of the bacteria is about 200 g/L, feeding is stopped, and the DO rises, indicating that the carbon source is exhausted and the bacteria enter an induction stage. An adjustment is performed so that pH=6.0±0.2, and expression of recombinant DF1 is induced with methanol at a feeding rate of 30 ml/h⁻¹L⁻¹. DO is maintained not higher than 40%, the induction temperature is maintained at 25-27°C, and pH=6.5±0.2, to perform fermentation expression. Samples are taken at regular intervals after the start of fermentation, and the OD₆₀₀ absorption value of the fermentation broth and the wet weight of the bacteria are measured. During the fermentation of recombinant *Pichia pastoris,* the OD₆₀₀ of the fermentation broth can reach 300 or more, and the wet weight of the bacteria can reach 400 g/L. The fermentation is terminated after induction for 40 h.

The above fermentation process of recombinant DP1 is also applicable to recombinant DF1.

### Example 3 Purification process of recombinant DP1/DF1

### I. First-step purification of recombinant DP1/DF1 fermentation broth supernatant

### Step 1: pretreatment of fermentation broth

The fermentation broth obtained in the above example is centrifuged at a high speed to obtain the supernatant. Diatomaceous earth is added to aid filtration, and a clarified fermentation broth sample is obtained overnight. The fermentation broth sample is diluted and ultrafiltrated with 10 KD membrane package to reduce the conductivity to 5 mS/cm or less.

### Step 2: cation exchange chromatography

The pH of the supernatant of the fermentation broth after the above treatment is adjusted with acetic acid to 4.0. The supernatant is loaded on an SP FF chromatography column, the column type is BPG140/100, and the column volume is 2800 ml. The equilibration buffer is 50 mM NaAc, pH 4.0. The elution buffer is 50 mM NaAc, 1.0 M NaCl, pH 4.0, and 0-100% linear elution is performed. The DP1/DF1 target proteins are mainly concentrated in the second elution peak.

Other conditions remain unchanged, and the pH of the sample and buffer is adjusted to 5.0.

Other conditions remain unchanged, and the pH of the sample and buffer is adjusted to 6.5.

After comparison, it is found that pH 5.0 is the best condition, and the purity of the DP1 protein is the highest after purification. In other conditions, impurities exist.

### II. Second-step purification of recombinant DP1/DF1 proteins:

### Step 1: ultrafiltration

The second elution peak in step 2 of the first-step purification above is mixed, and diluted to a conductivity of <2.0 mS/cm by 10 KD membrane-packed ultrafiltration.

### Step 2: anion exchange chromatography

20 mM Tris is added to the sample in step 1, and the pH is adjusted to 6.0. The sample is loaded on a Q FF anion exchange chromatography column, the column type is Hiscale 50/40, and the column bed volume is 500 ml. The equilibration buffer is 20 mMNaH₂PO₄, pH 6.0. The elution buffer is 20 mMNaH₂PO₄, 1.0 M NaCl, pH 6.0. The penetrant is collected, 0-100% linear elution is performed, and the target protein is mainly concentrated in the penetrant.

Other conditions remain unchanged, the pH of the sample and buffer is adjusted to 7.0, and the conductivity is 10.0 mS/cm.

Other conditions remain unchanged, the pH of the sample and buffer is adjusted to 8.0, and the conductivity is 20.0 mS/cm.

By comparison, it is found that the best condition is pH 8.0, conductivity 20.0 mS/cm. After optimization, the target protein has the highest degree of separation from impurities. Most of the target protein remains in the penetrant, and the impurities are bound to the medium, thereby achieving complete separation, and greatly improving the yield of the DP1/DF1 proteins in the penetrant.

### III. Third-step purification of recombinant DP1/DF1 proteins

Ammonium sulfate is added to the sample after the second-step purification to a final concentration of 1.0 M, and the pH is adjusted to 6.5. The sample is loaded on a Phenyl FF chromatography column, the column type is Hiscale 50/40, and the column bed volume is 500 ml. The equilibration buffer is 20 mM NaH₂PO₄, 1.0 M (NH₄)₂SO₄, pH 6.0. The elution buffer is 20 mM NaH₂PO₄, pH 6.0, and 25%, 50%, 70%, and 100% isocratic elution is performed.

Other conditions remain unchanged, and adjust ammonium sulfate in the sample and equilibration buffer to a concentration of 1.0 M, pH 7.5.

Other conditions remain unchanged, and adjust ammonium sulfate in the sample and equilibration buffer to a concentration of 2.0 M, pH 8.5.

By comparison, it is found that ammonium sulfate concentration 2.0 M and pH 8.5 are the optimal conditions. The optimized DP1/DF1 proteins have the highest purity and the least impurities, and the purity of the obtained protein can reach 95% or more.

The yield of the purification process shown in Table 1 after optimization is about 30% higher than that before optimization, and the activity (measured by ELISA method) is also improved compared to the level before process optimization.

**Table 1 Yields of DP1 protein before and after purification process optimization**

| Purification batch | Protein amount in supernatant of fermentation broth | Pure protein amount (mg) | Yield(%) |
|---|---|---|---|
| Before process optimization | 489 | 98 | 20 |
| First batch | 502 | 243 | 48 |
| Second batch | 471 | 247 | 52 |
| Third batch | 497 | 235 | 47 |

The above purification process of recombinant DP1 is also applicable to recombinant DF1.

### Example 4 Identification of recombinant DP1/DF1 proteins

### 1. Concentration and determination of protein concentration

The gradient elution peak in example 3 is collected and concentrated with a Vivaflow50 tangential flow ultrafiltration membrane package (VF05P9-50 cm², Sartorius). The buffer solution is replaced with PBS solution of pH 7.4 by dialysis bag. The protein concentration is determined with a Pierce BCA protein concentration kit.

### 2. HPLC purity analysis of DP1/DF1 proteins

The purified DP1/DF1 sample is diluted to 1 mg/ml and filtered with a 0.22 µm filter membrane to obtain the sample. The mobile phase is 20 mM PB of pH 7.5, the flow rate is 1 ml/min, and the injection volume is 10 µl. The purity is analyzed by an RP column. Figure 1-a and Figure 1-b show the purity test results. The purity of the DP1/DF1 proteins after three-step purification is greater than 95%.

### 3. Analysis of amino acid coverage of DP1/DF1 proteins

Amino acid coverage analysis is one of the most important indicators in the quality research of recombinant protein drugs. Only when the primary structures of amino acids are completely same, it can be ensured that the product has the same biological activity as the natural protein. The inventor entrusted Shanghai Applied Protein Technology Co., Ltd. to analyze the amino acid coverage of the DP1/DF1 proteins. The results of Figure 2-a and Figure 2-b show that the amino acid coverage is greater than 98% and meets the requirements of relevant policies and regulations.

### 4. Analysis of DP1/DF1 protein activity

Using a competitive inhibition ELISA method, the biological activity of recombinant DP1 and DF1 proteins (rDP1, rDF1) is measured and compared with that of natural DPI protein (nDP1). The specific steps (taking DP1 as an example) are as follows:
(1) Coating: rDP1 and nDP1 are diluted with a coating solution (pH 9.6, 0.15 M carbonate buffer) to 2 µg/ml respectively, and incubated as per 100 µl/well overnight at 4°C.
(2) Blocking: the rDP1 and nDP1 samples are washed with PBST (pH 7.4, 0.15 M PBS+0.05% Tween 20) 3 times, and pat dried. 200µl of blocking solution (1% BSA/PBST) is added to each well, and the rDP1 and nDP1 samples are incubated at 37°C for 2 h.
(3) Sample dilution: the rDP1 and nDP1 samples are diluted by 30 times, and then diluted by 3-time gradient with 7 dilutions.Samples of various dilutions and serum of appropriate dilutions in a serum bank are taken and mixed in equal volume, that is 125 µl sample + 125 µl serum.The serum bank is mixed with 15 or more copies of patient serum, the d1/d2 specific IgE value of which is >100 Kua/L detected by phadia100.The mixed samples are incubated overnight at 4°C. Positive control: 125 µl dilution + 125 µl serum.
(4) Sample addition: the incubated samples are mixed and added to the corresponding coating wells, and incubated at 37°C for 90 min.
(5) Secondary antibody incubation: the incubated samples are washed 4 times with PBST and pat dried. 100 µl of secondary antibody dilution (murine anti-human IgE-HRP, 1:1500 dilution) is added to each well, and the samples are incubated at 37°C for 1 h.
(6) Color development: the samples are washed 4 times with PBST and pat dried. 100 µl of TMB color development solution No. I is added to each well, and color development is performed at 37°C for 10 min.
(7) Termination and reading: 50 µl of 2 M H₂SO₄ is added to each well to terminate the reaction, and reading is performed at the wavelength of 450 nm.
(8) Result processing: EXCEL software is used, the inhibition rate% (positive value - sample value/positive value) is taken as the abscissa, and the Log10 dilution factor is taken as the ordinate to do a quadratic curve fitting. The 50% inhibition rate is substituted into the curve equation, and the dilution factor×100 is calculated as the biological activity value (BU/ml). Specific activity (BU/mg) = activity value/protein concentration.

The experimental results are shown in Table 2. The calculated activity value of rDP1 is 79616.0 BU/ml, and the specific activity is 9.95E+04 BU/mg. In the same way, nDF1 is tested as a control. The experimental results are shown in Table 2, and indicate that the rDP1 and rDF1 expressed by yeast have similar biological activity to natural protein.

**Table 2Comparison of activity values between recombinant protein and natural protein**

| Sample | 50% inhibition rate log10 | Dilution factor | Activity value (BU/mL) | Protein concentration (mg/mL) | Specific activity (BU/mg) |
|---|---|---|---|---|---|
| rDP1 | 2.901 | 796.1 | 79616.0 | 0.8 | 9.95E+04 |
| nDP1 | 2.718 | 552.4 | 55239.6 | 1.0 | 5.52E+04 |
| rDF1 | 2.513 | 325.8 | 32583.7 | 1.6 | 2.04E+04 |
| nDF1 | 2.316 | 207.0 | 20701.4 | 1.5 | 1.38E+04 |

### 5. Detection of host protein content in DP1/DF1

Host Cell Protein (HCP) refers to the host protein remaining in biological products. Such protein has complex components and many types, and changes due to different production process and purification process. Residual HCP in genetically engineered products is an important factor affecting the purity of products. Repeated use of genetically engineered products containing HCP can cause allergic reactions in the body, has a potential "adjuvant effect", and may also cause the body to produce antibodies to a drug, further affecting the efficacy of the drug. The residual amount of HCP in biological products reflects not only the consistency between batches of products, but also an important indicator to measure the quality of biological products. The inventor used a *Pichia pastoris* HCP detection kit (F140, CYGNUS) to detect the prepared samples. Table 3 shows that the HCP content of DP1 and DF1 proteins after three-step purification are far lower than the maximum HCP limit of recombinant biological products (yeast) stipulated in Chinese Pharmacopoeia 2015.

**Table 3 HCP detection results of DP1 and DF1 proteins**

| **Sample name** | **Purification step** | **Detection result (%)** | **Standard in Chinese Pharmacopoeia 2015 (%)** |
|---|---|---|---|
| DP1 | Three-step purification | 0.0051 | <0.1 |
| DF1 | Three-step purification | 0.0070 | <0.1 |

### 6. Detection of residual DNA content in DP1/DF1

Although recombinant protein drugs have been purified through a plurality of steps, there may still be DNA fragments of host cells remaining in the products. These residual DNA may bring infectious or tumorigenic risks, and may cause insertion mutations, inactivation of cancer suppressor genes, activation of oncogenes, etc. In addition, genomic DNA derived from microorganisms is rich in CpG and unmethylated sequences, which increases the risk of immunogenicity of recombinant protein drugs in the body. Therefore, WHO and drug registration regulatory agencies in various countries have strict requirements on the limit of residual DNA. The current methods for detecting the DNA residual amount mainly include DNA probe hybridization method, fluorescent dye method and qPCR method, wherein the first two methods have technical defects and are difficult to achieve the sensitivity for impurity limit detection. The FDA stipulated in the latest version of USP that qPCR is the only recommended method for detecting residual DNA. Therefore, the inventor used qPCR to detect residual DNA in the sample (SK030205P100, HuzhouShenke Biotechnology Co., Ltd.). The results in Table 4 show that the residual DNA content in the samples after the three-step purification is far below the maximum limit of the residual DNA content in recombinant biological products (yeast) stipulated in the Chinese Pharmacopoeia 2015.

**Table 4 Detection results of residual DNA in DP1 and DF1 proteins**

| **Sample name** | **Calculated value of DNA residual amount** | **Purification step** | **DNA content per mg of protein (pg)** | **Standard in Chinese Pharmacopoeia 2015 (ng)** |
|---|---|---|---|---|
| DP1 | 0.06 | Three-step purification | 7.36 | <10 |
| DF1 | 0.08 | Three-step purification | 5.57 | <10 |

### Example 5 Large-scale (30 L) high-density fermentation experiment of recombinant DP2/DF2

First, large-scale (30 L) high-density fermentation of DP2 is explained as follows.

### Step 1: activation of recombinant strain

Glycerol bacterial seeds obtained by example 1 and cryopreserved in a working seed bank at -80°C are taken and streaked on a YPD solid medium (yeast extract 10 g/L, peptone 20 g/L, glucose 20 g/L, and agarose 15 g/L), and cultured in a constant temperature and constant humidity box at 30°C for 3-5 days.

### Step 2: primary seed solution culture

The monoclonal colonies on the solid medium in step 1 are picked and cultured in a YPD liquid medium (yeast extract 10 g/L, peptone 20 g/L, and glucose 20 g/L) at 30°C and 220 rpm to OD₆₀₀≈6.0. The bacteria are observed under a microscope to ensure that there is no other bacteria, thus, the primary seed solution for fermentation is obtained.

### Step 3: secondary seed solution culture

The primary seed solution obtained in step 2 is inoculated into a Sartorius B-plus fermentation tank, and incubated in 60% BSM, at pH=5.5±0.2, 27°C, 300 rpm. Through the control of ventilation and rotation speed conditions, the dissolved oxygen is kept at 25%, until finally OD₆₀₀≈40, and the wet weight reaches about 80 g/L. The bacteria are observed under a microscope to ensure that there is no other bacteria, thus, the secondary seed solution for fermentation is obtained.

### Step 4: fermentation process

A Sartorius Cplus bioreactor is cleaned. A pH meter probe of the fermentation tank is calibrated with standard solutions of pH=7.0 and pH=4.0 respectively. Then, according to the designed interval of key process parameters in example 1, it is determined that the fermentation medium is 60% BSM, and 20 L of fermentation medium is prepared and poured into the fermentation tank. The fermentation medium is sterilized online at 121°C for 20 min. After the temperature drops to 50°C, an adjustment is performed with concentrated ammonia so that pH=5.5±0.2.

The seed solution obtained in step 3 is inoculated into the fermentation tank at a ratio of 1:15 (V/V, seed solution/fermentation medium). At the initial proliferation stage of recombinant *Pichia pastoris,* the fermentation temperature is 27°C, pH=6.5±0.2, the rotation speed is 300 rpm, the DO value is 100%, and PTM1 (2 ml/L) is added. The initial proliferation stage lasts for about 20 h. When the carbon source is exhausted, the dissolved oxygen value rises rapidly, and the wet weight of the bacteria reaches 100 g/L. At this time, the bacteria enter a rate-limited growth stage. In the first 2 h of the stage, 50% glycerol is added at a rate of 30 ml/h⁻¹L⁻¹. After 2 h, the feeding rate is increased to 60 ml/h⁻¹L⁻¹. After feeding for 4 h, the wet weight of the bacteria is about 200 g/L, feeding is stopped, and the DO rises, indicating that the carbon source is exhausted and the bacteria enter an induction stage. An adjustment is performed so that pH=6.5±0.2, and expression of recombinant DP2 is induced with methanol at a feeding rate of 30 ml/h⁻¹L⁻¹. DO is maintained not higher than 40%, the induction temperature is maintained at 20-22°C, and pH=6.0±0.2, to perform fermentation expression. Samples are taken at regular intervals after the start of fermentation, and the OD₆₀₀ absorption value of the fermentation broth and the wet weight of the bacteria are measured. During the fermentation of recombinant *Pichia pastoris,* the OD₆₀₀ of the fermentation broth can reach 300 or more, and the wet weight of the bacteria can reach 400%. The fermentation is terminated after induction for 40 h.

The above fermentation process of recombinant DP2 is also applicable to recombinant DF2.

### Example 6 Purification process of recombinant DP2/DF2

### I. First-step purification of recombinant DP2 fermentation broth supernatant

### Step 1. Pretreatment of fermentation broth

The fermentation broth obtained in the above example is centrifuged at a high speed to obtain the supernatant. Diatomaceous earth is added to aid filtration, and a clarified fermentation broth sample is obtained overnight. The fermentation broth sample is diluted and ultrafiltrated with 3 KD membrane-package to reduce the conductivity to 5 mS/cm or less.

### Step 2. Cation exchange chromatography

The pH of the supernatant of the fermentation broth after the above treatment is adjusted with acetic acid to 4.0. The supernatant is loaded on an SP FF chromatography column, the column type is BPG140/100, and the column volume is 2800 ml. The equilibration buffer is 50 mM NaAc, pH 4.0. The elution buffer is 50 mM NaAc, 1.0 M NaCl, pH 4.0, and 0-100% linear elution is performed. The target DP2/DF2 proteins are mainly concentrated in the second elution peak.

Other conditions remain unchanged, and the pH of the sample and buffer is adjusted to 5.0.

Other conditions remain unchanged, and the pH of the sample and buffer is adjusted to 6.0.

By comparison, it is found that as the pH increases, the obtained DP2 protein has a higher purity and fewer impurities, which is conducive to the subsequent purification treatment, wherein pH 6.0 is the best condition.

### II. Second-step purification of recombinant DP2 protein

### Step 1. Ultrafiltration

The second elution peak in step 2 of the example is mixed, and diluted to a conductivity of <2.0 mS/cm by 3 KD membrane-packed ultrafiltration.

### Step 2. Second-step anion exchange chromatography

20 mM Tris is added to the sample in step 1, and the pH is adjusted to 6.0. The sample is loaded on a Q FF anion exchange chromatography column, the column type is Hiscale 50/40, and the column bed volume is 500 ml. The equilibration buffer is 20 mM NaH₂PO₄, pH 6.0. The elution buffer is 20 mM NaH₂PO₄, 1.0 M NaCl, pH 6.0. The penetrant is collected, 0-100% linear elution is performed, and the target protein is mainly concentrated in the penetrant.

Other conditions remain unchanged, the pH of the sample and buffer is adjusted to 7.5, and the conductivity is 10.0 mS/cm.

Other conditions remain unchanged, the pH of the sample and buffer is adjusted to 9.0, and the conductivity is 20.0 mS/cm.

By comparison, it is found that the best condition is pH 7.5, conductivity 10.0 mS/cm. After optimization, the target protein has a higher degree of separation from impurities. Most of the target protein remains in the penetrant, and the impurities are bound to the medium, thereby achieving complete separation, and greatly improving the yield of the DP2 protein in the penetrant.

### III. Third-step purification of recombinant DP2 protein

Ammonium sulfate is added to the sample after the second-step purification to a final concentration of 1.0 M, and the pH is adjusted to 6.0. The sample is loaded on a Phenyl FF chromatography column, the column type is Hiscale 50/40, and the column bed volume is 500 ml. The equilibration buffer is 20 mMNaH₂PO₄, 1.0 M (NH₄)₂SO₄, pH 6.0. The elution buffer is 20 mM NaH₂PO₄, pH 6.0, and 25%, 50%, 70%, and 100% isocratic elution is performed.

Other conditions remain unchanged, and adjust ammonium sulfate in the sample and equilibration buffer to a concentration of 1.0 M, pH 7.0.

Other conditions remain unchanged, and adjust ammonium sulfate in the sample and equilibration buffer to a concentration of 2.0 M, pH 8.0.

By comparison, it is found that the best condition is ammonium sulfate concentration 1.0 M and pH 6.0. The optimized DP2 protein has a higher purity and the amount of protein is the largest. There is no target protein in the penetrant, and all the target proteins are bound to packing.

As shown in Table 5, the purification yield of DP2 protein after optimization is 30% higher than that before optimization, and the activity (measured by ELISA method) is also improved compared with that before process optimization.

**Table 5 Yields of DP2 protein before and after purification process optimization**

| Purification batch | Protein amount in supernatant of fermentation broth | Pure protein amount (mg) | Yield(%) |
|---|---|---|---|
| Before process optimization | 217 | 43 | 20 |
| First batch | 209 | 102 | 49 |
| Second batch | 225 | 106 | 47 |
| Third batch | 211 | 116 | 55 |

The above purification process of recombinant DP2 is also applicable to recombinant DF2.

### Example 7 Identification of recombinant DP2/DF2 proteins

### 1. Concentration and determination of protein concentration

The gradient elution peak in example 6 is collected and concentrated with a Vivaflow50 tangential flow ultrafiltration membrane package (VF05P9-50 cm², Sartorius). The buffer solution is replaced with PBS solution of pH 7.4 by dialysis bag. The protein concentration is determined with a Pierce BCA protein concentration kit.

### 2. HPLC purity analysis of DP2/DF2 proteins

The purified DP2/DF2 sample is diluted to 1 mg/ml and filtered with a 0.22 µm filter membrane to obtain the sample. The mobile phase is 20 mM PB of pH 7.5, the flow rate is 1 ml/min, and the injection volume is 10 µl. The purity is analyzed by an RP column. Figure 3-a and Figure 3-b show the purity test results. The purity of the DP2/DF2 proteins after three-step purification is greater than 95%.

### 3. Analysis of amino acid coverage of DP2/DF2 proteins

Amino acid coverage analysis is one of the most important indicators in the quality research of recombinant protein drugs. Only when the primary structures of amino acids are completely same, it can be ensured that the product has the same biological activity as the natural protein. The inventor entrusted Shanghai Applied Protein Technology Co., Ltd. to analyze the amino acid coverage of the DP2/DF2 proteins. The results of Figure 4-a and Figure 4-b show that the amino acid coverage is 100% and meets the requirements of relevant policies and regulations.

### 4. Detection of host protein content in DP2/DF2

Host Cell Protein (HCP) refers to the host protein remaining in biological products. Such protein has complex components and many types, and changes due to different production process and purification process. Residual HCP in genetically engineered products is an important factor affecting the purity of products. Repeated use of genetically engineered products containing HCP can cause allergic reactions in the body, has a potential "adjuvant effect", and may also cause the body to produce antibodies to a drug, further affecting the efficacy of the drug. The residual amount of HCP in biological products reflects not only the consistency between batches of products, but also an important indicator to measure the quality of biological products. The inventor used a *Pichia pastoris* HCP detection kit (F140, CYGNUS) to detect the prepared samples. Table 6 shows that the HCP content of DP2 and DF2 proteins after three-step purification are far lower than the maximum HCP limit of recombinant biological products (yeast) stipulated in Chinese Pharmacopoeia 2015.

**Table 6 HCP detection results of DP2 and DF2 proteins**

| **Sample name** | **Purification step** | **Detection result (%)** | **Standard in Chinese Pharmacopoeia 2015 (%)** |
|---|---|---|---|
| DP2 | Three-step purification | 0.0025 | <0.1 |
| DF2 | Three-step purification | 0.0100 | <0.1 |

### 5. Detection of residual DNA content in DP2/DF2

Although recombinant protein drugs have been purified through a plurality of steps, there may still be DNA fragments of host cells remaining in the products. These residual DNA may bring infectious or tumorigenic risks, and may cause insertion mutations, inactivation of cancer suppressor genes, activation of oncogenes, etc. In addition, genomic DNA derived from microorganisms is rich in CpG and unmethylated sequences, which increases the risk of immunogenicity of recombinant protein drugs in the body. Therefore, WHO and drug registration regulatory agencies in various countries have strict requirements on the limit of residual DNA. The current methods for detecting the DNA residual amount mainly include DNA probe hybridization method, fluorescent dye method and qPCR method, wherein the first two methods have technical defects and are difficult to achieve the sensitivity for impurity limit detection. The FDA stipulated in the latest version of USP that qPCR is the only recommended method for detecting residual DNA. Therefore, the inventor used qPCR to detect residual DNA in the sample (SK030205P100, HuzhouShenke Biotechnology Co., Ltd.). The results in Table 7 show that the residual DNA content in the samples after the three-step purification is far below the maximum limit of the residual DNA content in recombinant biological products (yeast) stipulated in the Chinese Pharmacopoeia 2015.

**Table 7 Detection results of residual DNA in DP2 and DF2 proteins**

| **Sample name** | **Calculated value of DNA residual amount** | **Purification step** | **DNA content per mg of protein (pg)** | **Standard in Chinese Pharmacopoeia 2015 (ng)** |
|---|---|---|---|---|
| DP2 | 0.05 | Three-step purification | 6.73 | <10 |
| DF2 | 0.09 | Three-step purification | 5.75 | <10 |

### Example 8 Comparison of activity between single recombinant protein and natural protein

Using a competitive inhibition ELISA method, the biological activity of rDF1/rDF2/rDP1/rDP2 is measured and compared with that of natural nDF1/nDF2/nDP1/nDP2. The specific steps (taking rDP2 as an example) are as follows:
1. Coating: rDP2/nDP2 are diluted with a coating solution (pH 9.6, 0.15 M carbonate buffer) to 2 µg/ml respectively, and incubated as per 100 µl/well overnight at 4°C.
2. Blocking: the rDP2 and nDP2 samples are washed with PBST (pH 7.4, 0.15 M PBS+0.05% Tween 20) 3 times, and pat dried. 200 µl of blocking solution (1% BSA/PBST) is added to each well, and the rDP2 and nDP2 samples are incubated at 37°C for 2 h.
3. Sample dilution: the rDP2/nDP2 samples are diluted by 1000 times, and then diluted by 3-time gradient with 7 dilutions. Samples of various dilutions and serum of appropriate dilutions in a serum bank are taken and mixed in equal volume, that is 125 µl sample + 125 µl serum. The serum bank is mixed with 15 or more copies of patient serum, the dl/d2 specific IgE value of which is >100 Kua/L detected by phadia100.The mixed samples are incubated overnight at 4°C. Positive control: 125 µl dilution + 125 µl serum.
4. Sample addition: the incubated samples are mixed and added to the corresponding coating wells, and incubated at 37°C for 90 min.
5. Secondary antibody incubation: the incubated samples are washed 4 times with PBST and pat dried. 100 µl of secondary antibody dilution (murine anti-human IgE-HRP, 1:1500 dilution) is added to each well, and the samples are incubated at 37°C for 1 h.
6. Color development: the samples are washed 4 times with PBST and pat dried. 100 µl of TMB color development solution No. I is added to each well, and color development is performed at 37°C for 10 min.
7. Termination and reading: 50 µl of 2 M H₂SO₄ is added to each well to terminate the reaction, and reading is performed at the wavelength of 450 nm.
8. Result analysis: EXCEL software is used, the inhibition rate% (positive value - sample value/positive value) is taken as the abscissa, and the LoglO dilution factor is taken as the ordinate to do a quadratic curve fitting. The 50% inhibition rate is substituted into the curve equation, and the dilution factor×100 is calculated as the biological activity value (BU/ml). Specific activity (BU/mg) = activity value/protein concentration. The experimental results are shown in Table 8 and Figure 5. The calculated activity value of rDP2 is 125675 BU/ml, and the specific activity is 1.40E+05 BU/mg. In the same way, nDF1/nDF2/nDP1/nDP2 are tested as a control. The experimental results are shown in Table 8, and indicate that the rDF1/rDF2/rDP1/rDP2 expressed by yeast have similar biological activity to natural protein.

**Table 8 Comparison of activity values between recombinant protein and natural protein**

| Sample | 50% inhibition rate log 10 | Dilution factor | Activity value (BU/ml) | Protein concentration (mg/ml) | Specific activity (BU/mg) |
|---|---|---|---|---|---|
| rDP1 | 5.17 | 146808.10 | 14680809.4 | 1.1 | 1.29E+07 |
| nDP1 | 4.73 | 53119.02 | 5311901.3 | 1.10 | 4.83E+06 |
| rDF1 | 5.03 | 107398.94 | 10739894.1 | 1.56 | 6.88E+06 |
| nDF1 | 5.17 | 148679.17 | 14867912.6 | 1.00 | 1.49E+07 |
| rDP2 | 4.17 | 14859.32 | 1485935.6 | 1.00 | 1.49E+06 |
| nDP2 | 4.25 | 17762.30 | 1776233.3 | 1.80 | 9.87E+05 |
| rDF2 | 3.68 | 4830.64 | 483058.8 | 1.5 | 3.31E+05 |
| nDF2 | 3.41 | 2588.28 | 258821.3 | 1.20 | 2.16E+05 |

### Example 9 Comparison of activity between recombinant mixture (equal mass) and natural mixture (equal mass)

1. Coating and dilution are performed as follows:

**Table 9**

| Coating group | Natural mixture(nDP1, nDP2, nDF1, nDF2) | Recombinant mixture(rDP1, rDP2, rDF1, rDF2) |
|---|---|---|
| Coating content | 0.5 µg respectively | 0.5 µg respectively |
| Diluted sample type and content | Natural mixture(nDP1, nDP2, nDFl, nDF2) 0.5 µgrespectively | Recombinant mixture(rDP1, rDP2, rDFl, rDF2) 0.5 µgrespectively |

2. During dilution, the recombinant and natural mixture samples are mixed in equal mass (1:1:1:1) into a mixture of 1.0 mg/ml respectively. The mixture is diluted by 1000 times to the actual measured concentration, and diluted by 3 time dilution with 7 dilutions.for 7 gradients down. The recombinant mixture and the natural mixture diluted sample and serum are mixed and then added to a coating system coated with the natural mixture for detection.
3. The rest of the operation steps are basically the same as in example 8.
4. Result analysis: the results in Table 10 show that compared with the natural mixture, the recombinant mixture has a higher in-vitro activity and higher binding force with serum IgE.

**Table 10 Comparison of activity between recombinant mixture and natural mixture**

| Sample | 50%inhibition rate log10 | Dilution factor | Activity value (BU/ml) | Protein concentration (mg/ml) | Specific activity (BU/mg) |
|---|---|---|---|---|---|
| Natural mixture | 3.55 | 3552.22 | 355222.15 | 1.14 | 3.55E+05 |
| Recombinant mixture | 4.17 | 14769.81 | 1476981.27 | 1.10 | 1.48E+06 |

### Example 10 Comparison of activity between recombinant mixture and single component recombinant protein

Using the competitive inhibition ELISA method, the recombinant mixture is mixed according to the following ratio and compared with a single component of rDF1/rDF2/rDP1/rDP2 in biological activity. The specific steps (taking rDP1 as an example) are as follows:
1. Coating: a recombinant mixture is prepared according to a mass ratio of rDP1:rDP2:rDF1:rDF2=20:1:20:1, diluted to 2 µg/ml, and incubated as per 100 µl/well overnight at 4°C. (The ratio of components in other recombinant mixtures is in the same order)
2. Blocking: the recombinant mixture is washed with PBST (pH 7.4, 0.15 M PBS+0.05% Tween 20) 3 times, and pat dried. 200 µl of blocking solution (1% BSA/PBST) is added to each well, and the recombinant mixture is incubated at 37°C for 2 h.
3. Sample dilution: the recombinant mixture mixed in step 1 and a comparative rDP1 sample are diluted by 1000 times, and then diluted by 3-time gradient dilution with 7 dilutions. Samples of various dilutions and serum of appropriate dilutions in a serum bank are taken and mixed in equal volume, that is 125 µl sample + 125 µl serum. The serum bank is mixed with 15 or more copies of patient serum, the d1/d2 specific IgE value of which is >100 Kua/L detected by phadia100.The mixed samples are incubated overnight at 4°C. Positive control: 125 µl dilution + 125 µl serum.
4. The rest of the operation steps are basically the same as in example 8.
5. Result analysis: EXCEL software is used, the inhibition rate% (positive value - sample value/positive value) is taken as the abscissa, and the Log10 dilution factor is taken as the ordinate to do a quadratic curve fitting.The 50% inhibition rate is substituted into the curve equation, and the dilution factor×100 is calculated as the biological activity value (BU/ml). Specific activity (BU/mg) = activity value/protein concentration. The activity of the comparative rDP1 is defined as 100%, and the ratio of the activity of various comparative recombinant mixtures to rDP1 is calculated (the method for comparing the activity of the recombinant mixtures and the rDP2, rDF1 and rDF2 is the same as that of rDP1). The experimental results are shown in Figure 6, and the recombinant mixtures in a certain ratio show significantly higher activity than the single recombinant component.

### Example 11 Comparison of activity between recombinant mixture and type I and type II recombinant allergen proteins

Using the competitive inhibition ELISA method, the recombinant mixture is mixed according to the following ratio and compared with a single component of rDF1/rDF2/rDP1/rDP2 in biological activity. The specific steps (taking rDP1 as an example) are as follows:
1. Coating: a recombinant mixture is prepared according to a mass ratio of rDP1:rDP2:rDF1:rDF2=20:1:20:1, diluted to 2 µg/ml, and incubated as per 100 µl/well overnight at 4°C. (The ratio of components in other recombinant mixtures is in the same order)
2. Blocking: the recombinant mixture is washed with PBST (pH 7.4, 0.15 M PBS+0.05% Tween 20) 3 times, and pat dried. 200 µl of blocking solution (1% BSA/PBST) is added to each well, and the recombinant mixture is incubated at 37°C for 2 h.
3. Sample dilution: the recombinant mixture mixed in 1 and comparative rDP1+rDF1 and rDP2+rDP2 samples (mixed in ratios of 20:1, 5:1, 1:1, 1:5 and 1:20) are diluted by 1000 times, and then diluted by 3-time gradient with 7 dilutions. Samples of various dilutions and serum of appropriate dilutions in a serum bank are taken and mixed in equal volume, that is 125 µl sample + 125 µl serum. The serum bank is mixed with 15 or more copies of patient serum, the d1/d2 specific IgE value of which is >100 Kua/L detected by phadia100.The mixed samples are incubated overnight at 4°C. Positive control: 125 µl dilution + 125 µl serum.
4. The rest of the operation steps are basically the same as in example 8.
5. Result analysis: EXCEL software is used, the inhibition rate % (positive value - sample value/positive value) is taken as the abscissa, and the Log10 dilution factor is taken as the ordinate to do a quadratic curve fitting. The 50% inhibition rate is substituted into the curve equation, and the dilution factor×100 is calculated as the biological activity value (BU/ml). Specific activity (BU/mg) = activity value/protein concentration. The activity of the comparative rDP1 is defined as 100%, and the ratio of the activity of various comparative recombinant mixtures to rDP1 is calculated (the method for comparing the activity of the recombinant mixtures and the rDP2, rDF1 and rDF2 is the same as that of rDPl). The experimental results are shown in Figure 7, and the recombinant mixtures in almost all ratios show significantly higher activity than the type I and type II recombinant allergens. Similar to the reason of example 10, the synergistic effect of the 4 recombinant mixtures is greater than that of the type I or type II recombinant allergen alone.

### Example 12 Comparison of activity between recombinant mixture and Odactra

Using a competitive inhibition ELISA method, the activity of the recombinant mixture measured in example 8 is compared with the biological activity of Odactra.
1. Coating: a recombinant mixture is prepared according to an equal activity ratio of rDP1:rDP2:rDF1:rDF2=1:1:1:1, diluted to 2 µg/ml, and incubated as per 100 µl/well overnight at 4°C.
2. Blocking: the recombinant mixture is washed with PBST (pH 7.4, 0.15 M PBS+0.05% Tween 20) 3 times, and pat dried. 200 µl of blocking solution (1% BSA/PBST) is added to each well, and the recombinant mixture is incubated at 37°C for 2 h.
3. Sample dilution: the recombinant mixture mixed in 1 and a comparative Odactra sample (calculated according to a labeled amount of 30 µg/piece) are diluted by 1000 times, and then diluted by 3-time gradient with 7 dilutions. Samples of various dilutions and serum of appropriate dilutions in a serum bank are taken and mixed in equal volume, that is 125 µl sample + 125 µl serum. The serum bank is mixed with 15 or more copies of patient serum, the d1/d2 specific IgE value of which is >100 Kua/L detected by phadia100. The mixed samples are incubated overnight at 4°C. Positive control: 125 µl dilution + 125 µl serum.
4. The rest of the operation steps are basically the same as in example 8.
5. Result analysis: EXCEL software is used, the inhibition rate % (positive value - sample value/positive value) is taken as the abscissa, and the Log10 dilution factor is taken as the ordinate to do a quadratic curve fitting. The 50% inhibition rate is substituted into the curve equation, and the dilution factor×100 is calculated as the biological activity value (BU/ml). Specific activity (BU/mg) = activity value/protein concentration. The activity of the comparative Odactra is defined as 100%, and the ratio of the activity of the comparative recombinant mixture to the Odactra is calculated. The experimental results are shown in Figure 8, and the activity of the recombinant mixture with equal activity is 40% higher than that of Odactra, indicating that the activity of the recombinant mixture with equal activity is significantly higher than that of Odactra.

### Example 13 Comparison of desensitization effect between recombinant mixture and positive drug Odactra

1. Model preparation and desensitization treatment: BALB/c mice are intraperitoneal injected with HDM (mite extract, purchased from LSL, with DF 0.45 µgand DP 0.40 µg) + aluminum adjuvant, for about 4 consecutive weeks. After the last sensitization, HDM solution is instilled into the nasal cavity (with DF 0.90 µgand DP 0.80 µg) for 7 consecutive days. After the challenge is over, indicators are detected, including: sneeze, penh value(the animals are stimulated by methacholine Mch at the concentrations of 3.125/6.25/12.5/25/50 mg/ml respectively, using a whole body volume scanner WBP of BUXCO for detection) and the like. The animals are sorted in descending order according to the average penh value at each concentration, and the animals with higher values are selected into the group, and divided into a PBS treatment group, a recombinant mixture group (DP1, DF1, DP2 and DF2 in a mass ratio of 1:1:1:1), 25 µg/animal, and a positive group 24 µg/animal (based on the active components of Odactra). Sublingual desensitization treatment is performed 2 times a week (2-3 days between 2 desensitization treatments) for 8 consecutive weeks. The nasal cavity is stimulated again in the same way for 7 consecutive days. After the stimulation is over, indicators are detected, including: sneeze, penh value and spleen cytokines.
2. Result analysis: as shown in Table 11, the number of sneezes of mice in the recombinant mixture group, positive group and normal group is less than that of the PBS group, with significant difference (p<0.01). In addition, under the stimulation of Mch at a concentration of 3.125-50 mg/ml, the penh values of mice in the recombinant mixture group and the positive group are significantly lower than that of the PBS group, and the penh value of the recombinant mixture group and the positive group are close. As shown in Figure 9, after desensitization, the content of IL-4, IL-5, and IL-13 in the recombinant mixture group is lower than that in the positive group, and significantly decreases compared with the PBS group (P<0.05); and after desensitization, the IL-10 levels of the recombinant mixture group and the positive group are similar, and both show a significant decrease compared with the PBS group. It shows that the cytokine shifts from significant Th2 type to the balance between Th2 and Thl, and shows that the allergy of the model mice is improved on the cytokine level or desensitization mechanism. In summary, after the sublingual desensitization treatment, the recombinant mixture group and the positive group have significant improvement effects in respiratory symptoms and mechanisms such as the penh value, the number of sneezes and the cytokine level, showing that the recombinant mixture has the same activity in the animal in-vivo efficacy test.

**Table 11 Counting of Penh value and sneeze (mean±sem)**

| Group | Number of animals | Penh value at each Mch concentration (mg/ml) | | | | | Number of sneezes (times) |
|---|---|---|---|---|---|---|---|
| | | 3.125 | 6.25 | 12.5 | 25 | 50 | |
| PBS treatment group | 12 | 2.97±0.19 | 5.71±1.07 | 7.87±1.19 | 13.42±2.87 | 19.83±3.03 | 29.36±3.59 |
| Recombinant mixture group | 12 | 2.72±0.26 | 4.40±0.74 | 5.17±0.75 | 6.26±1.19 | 16.34±1.55 | 15.58±1.7 |
| Positive group | 8 | 2.46±0.53 | 4.59±1.93 | 4.57±0.4 | 6.51±1.28 | 17.79±2.51 | 18.78±2.92 |
| Normal group | 8 | 3.31±0.98 | 4.04±0.77 | 4.89±0.77 | 6.33±1.11 | 10.4±1.87 | 11.07±2.64 |

## Claims

1. A recombinant dust mite allergen protein pharmaceutical composition, wherein the pharmaceutical composition comprises recombinant dust mite type I allergen DP1 and DF1 proteins and recombinant dust mite type II allergen DP21 and DF2 proteins, and the four types of dust mite allergen proteins DP1 :DP2:DF1 :DF2 are respectively mixed in an activity ratio of 1:1:1:1 or mixed in a mass ratio of (1-5):(1-20):(1-10):(1-20).

2. The recombinant dust mite allergen protein pharmaceutical composition according to claim 1, wherein the four types of dust mite allergen proteins DP1:DP2:DF1:DF2 in the pharmaceutical composition are respectively mixed in a mass ratio of 1:(1-20):1:(1-20).

3. The recombinant dust mite allergen protein pharmaceutical composition according to claim 1, wherein the four types of dust mite allergen proteins DP1:DP2:DF1:DF2 in the pharmaceutical composition are respectively mixed in a mass ratio of 1:5:1:5.

4. The recombinant dust mite allergen protein pharmaceutical composition according to any one of claims 1-3, wherein the recombinant dust mite type I allergen DP1 and DF1 proteins and the recombinant dust mite type II allergen DP2 and DF2 proteins are respectively obtained by expressing the genes as shown in SEQ ID NO: 1, SEQ ID NO: 2, SEQ ID NO: 3 and SEQ ID NO: 4 through a *Pichia pastoris* expression system.

5. The recombinant dust mite allergen protein pharmaceutical composition according to claim 4, wherein the recombinant dust mite type I allergen protein is prepared by the following method:
firstly, the gene of claim 4 is constructed on a plasmid pPICZα, the plasmid is transfected into *Pichia pastoris* X33 competent cells, and the cells are cultured to obtain the host monoclonal engineered bacteria; and secondly, the monoclonal engineered bacteria are selected and subjected to high-density fermentation, and the recombinant dust mite type I allergen protein is obtained by three steps of purification of ion exchange chromatography, anion exchange chromatography, and hydrophobic chromatography.

6. The recombinant dust mite allergen protein pharmaceutical composition according to claim 5, wherein according to the preparation method, the main fermentation process for expressing the recombinant dust mite type I allergen DP1 and DF1 proteins is as follows: the fermentation medium used is a 40% BSM medium; at the induction stage of high-density fermentation, the induction temperature is 25-27°C, and the pH value is 6.5±0.2; at the induction stage of high-density fermentation, the feeding rate of methanol is 30 mL/h⁻¹L⁻¹, and DO is maintained not higher than 40%; at the proliferation stage of bacteria during high-density fermentation, the fermentation temperature is 27°C, the pH is 5.5±0.2, the rotation speed is 300 rpm, the DO value is 100%, and PTM1 (2.4 ml/L) is added; and at the start of the rate-limited growth stage of high-density fermentation, 50% glycerol is fed at a rate of 30 ml/h⁻¹L⁻¹, and then the feeding rate is increased to 60 ml/h⁻¹L⁻¹.

7. The recombinant dust mite allergen protein pharmaceutical composition according to claim 5, wherein according to the preparation method, the main fermentation process for expressing the recombinant dust mite type II allergen DP2 and DF2 proteins is as follows: the used fermentation medium is a 60% BSM medium; at the induction stage of high-density fermentation, the induction temperature is 20-22°C, and the pH value is 6.0±0.2; at the induction stage of high-density fermentation, the feeding rate of methanol is 30 mL/h⁻¹L⁻¹, and DO is maintained not higher than 40%; and at the start of the rate-limited growth stage of high-density fermentation, 50% glycerol is fed at a rate of 30 ml/h⁻¹L⁻¹, and then the feeding rate is increased to 60 ml/h⁻¹L⁻¹.

8. The recombinant dust mite allergen protein pharmaceutical composition according to claim 5, wherein according to the preparation method, when the recombinant dust mite type I allergen DP1 and DF1 proteins are purified,
the purification packing for cation exchange chromatography is SP FF, and the pH is 5.0;
the purification packing for the anion exchange chromatography is Q FF, the pH is 8.0, and the conductivity is 20.0 mS/cm; and
the purification packing for the hydrophobic chromatography is phenyl FF, the concentration of ammonium sulfate in the sample and equilibration buffer is 2.0 M, and the pH is 8.5.

9. The recombinant dust mite allergen protein pharmaceutical composition according to claim 5, wherein according to the preparation method, when the recombinant dust mite type II allergen DP2 and DF2 proteins are purified,
the purification packing for cation exchange chromatography is SP FF, and the pH is 6.0;
the purification packing for the anion exchange chromatography is Q FF, the pH is 7.5, and the conductivity is 10.0 mS/cm; and
the purification packing for the hydrophobic chromatography is phenyl FF, the concentration of ammonium sulfate in the sample and equilibration buffer is 1.0 M, and the pH is 6.0.
